(19) Europäisches Patentamt
European Patent Office
Office européen des brevets

(11) Numéro de publication : **0 133 407**
**B1**

(12) # FASCICULE DE BREVET EUROPÉEN

(45) Date de publication du fascicule du brevet : **08.07.87**

(21) Numéro de dépôt : **84401627.9**

(22) Date de dépôt : **03.08.84**

(51) Int. Cl.[4] : **C 07 C 95/02**, C 07 C 95/06, C 07 C 93/10, C 07 C 93/12, C 07 C 97/02, C 07 C 97/065, C 07 C103/187// C07C101/40, C07F7/10

(54) **Dérivés acétyléniques possédant une activité d'inhibiteurs d'enzyme, leur préparation et leur application comme médicament.**

(30) Priorité : **04.08.83 FR 8312863**

(43) Date de publication de la demande : **20.02.85 Bulletin 85/08**

(45) Mention de la délivrance du brevet : **08.07.87 Bulletin 87/28**

(84) Etats contractants désignés : **AT BE CH DE FR GB IT LI LU NL SE**

(56) Documents cités :
**DE-A- 1 568 274**
**DE-A- 1 768 331**
**CHEMICAL ABSTRACTS, vol. 77, no. 3, 17 juillet 1972, page 497, no. 19497d, Columbus, Ohio, US; B.P. GUSEV et al.: "Chemistry of polyene and polyacetylene compounds. XXVII. Behavior of 5-amino-5-methylhexa-1,3-diyne in addition reactions" & KHIM. GETEROTSIKL. SOEDIN. 1971, 7(11), 1530-2**

(73) Titulaire : **Etablissement Public dit: CENTRE NATIONAL DE LA RECHERCHE SCIENTIFIQUE (CNRS)**
**15, Quai Anatole France**
**F-75007 Paris (FR)**

**INSTITUT NATIONAL DE LA SANTE ET DE LA RECHERCHE MEDICALE (INSERM)**
**101, rue de Tolbiac**
**F-75654 Paris Cedex 13 (FR)**

(72) Inventeur : **Doutheau, Alain Guy**
**75 cours de la République**
**F-69100 Villeurbanne (FR)**
Inventeur : **Gore, Jacques**
**7, rue de Mont Cindre**
**F-69300 Caluire (FR)**
Inventeur : **Quash, Gérard**
**Village de l'Ouest 12 Allée des Charmilles**
**F-69340 Francheville (FR)**

(74) Mandataire : **Nony, Michel**
**Cabinet Nony 29, rue Cambacérès**
**F-75008 Paris (FR)**

**EP 0 133 407 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet de nouveaux dérivés acétyléniques possédant une activité d'inhibiteurs d'enzyme, leur préparation et leur application comme médicament.

L'invention a plus précisément pour objet les composés de formule générale I :

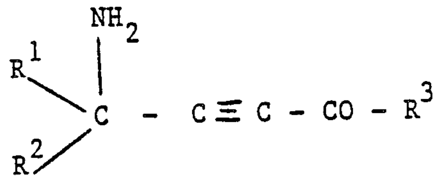

(I)

dans laquelle $R^1$ et $R^2$ représentent chacun indépendamment un groupement alkyle ayant de 1 à 5 atomes de carbone, ou bien $R^1$ et $R^2$ représentent ensemble un groupement alkylène ayant 4 ou 5 atomes de carbone, et $R^3$ représente —H, un groupement alkyle inférieur ou un groupement $—NH_2$ ; ainsi que les cétals correspondants et les sels d'addition des composés de formule I, et les solutions contenant lesdits composés ou sels d'addition ; à l'exception du composé de formule I pour lequel $R^1 = R^2 = CH_3$ et $R^3 = CH_3$.

On connaissait déjà, par l'article de B.P. Gusev et al., Khimia Geterot Siklicheskikh Soedinenii, n° 11, pp. 1530-1532 (1971), un produit qui correspondrait à la formule I donnée ci-dessus, avec $R^1 = R^2 = R^3 = CH_3$, ce produit étant décrit uniquement comme intermédiaire dans la préparation de certains hétérocycles.

Parmi les composés de formule I on citera notamment ceux pour lesquels :

$R^3$ représente un groupement alkyle ayant 1 à 5 atomes de carbone ;

$R^3$ est choisi parmi les groupements —H, $—CH_3$ et $—NH_2$ ;

$R^1 = R^2 = CH_3$ et $R^3 = H$ ;

$R^1 = R^2 = CH_3$ et $R^3 = NH_2$ ;

$R^1 = CH_3$, $R^2 = C_2H_5$ et $R^3 = H$ ;

$R^1 = R^3 = CH_3$ et $R^2 = C_2H_5$ ; ou

$R^1 + R^2 = —(CH_2)_5—$ et $R^3 = H$ ; ainsi que les cétals et les sels d'addition de ces composés.

Les cétals sont notamment les dialkyl cétals de formule :

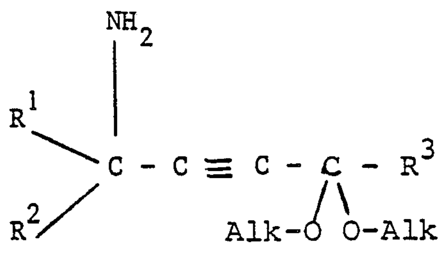

où Alk est un alkyle inférieur.

Les cétals peuvent être considérés comme des précurseurs stables des composés de formule I correspondants.

Les sels d'addition des composés de formule I sont notamment ceux formés avec les acides compatibles avec une utilisation en thérapeutique.

L'invention a également pour objet le procédé de préparation des composés de formule I.

Ce procédé est caractérisé par le fait que l'on utilise comme produit de départ un composé de formule II :

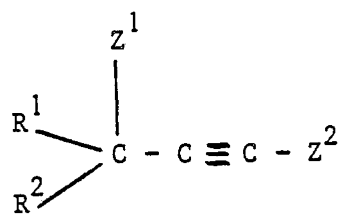

(II)

dans laquelle $R^1$ et $R^2$ sont définis comme précédemment, $Z^1$ représente un groupement amine protégé (la liaison $C—Z^1$ étant une liaison carbone-azote), et $Z^2$ représente soit —H soit un équivalent d'un métal ou d'un groupement lié à l'atome de carbone auquel il est attaché par une liaison carbone-métal, que l'on

fait réagir ledit composé de départ avec un agent d'acylation ou d'alkoxycarbonylation, pour obtenir un composé de formule III :

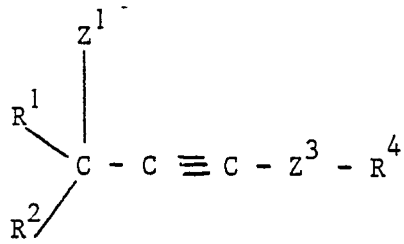

(III)

dans laquelle $R^1$, $R^2$ et $Z^1$ sont définis comme précédemment, $R^4$ représente —H, alkyle inférieur et $Z^3$ représente soit un groupement carbonyle —CO—, soit un groupement précurseur du groupement carbonyle, ou $R^4$ représente un alkoxy inférieur et $Z^3$ représente un groupement —CO—, puis que l'on soumet le composé obtenu à une réaction de déprotection du groupement amine et que, le cas échéant, soit on transforme, si désiré, le groupement —$Z^3$— en groupement carbonyle, soit, dans le cas où —$Z^3$—$R^4$ représente un groupement alkoxycarbonyle, on transforme l'ester obtenu en amide correspondant, et on obtient le composé de formule I désiré ou un sel d'addition correspondant que l'on transforme, si désiré, en cétal et/ou en sel d'addition correspondant.

Les produits de départ sont obtenus par réaction avec un groupement protecteur usuel des produits de formule IV :

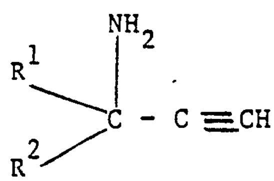

(IV)

Les produits de formule IV sont connus ou peuvent être préparés selon les méthodes connues, par exemple selon la méthode décrite par G.F. Hennion et E.G. Teach, J. Org. Chem. 1952. 17, 1653.

Pour la protection de l'amine primaire du composé de formule IV, et pour la déprotection ultérieure, on peut utiliser par exemple une méthode analogue à celle décrite par S. Djuric, J. Venit et P. Magnus, Tetrahedron Letters, 1981, 1787. Cette méthode consiste à faire réagir l'amine de formule IV avec un composé de formule V :

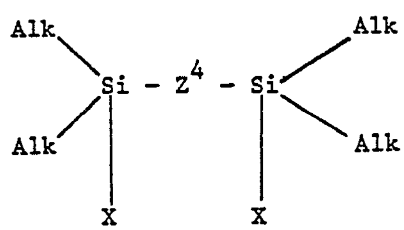

(V)

dans laquelle Alk est un alkyle inférieur, $Z^4$ est un groupement alkylène et X est un atome d'halogène. La réaction de déprotection de l'amine s'effectue par hydrolyse acide, par exemple par contact avec la silice humide.

Le produit obtenu après la réaction de protection est caractérisé par le fait que $Z^1$ (notamment dans les formules II et III ci-dessus) représente un groupement :

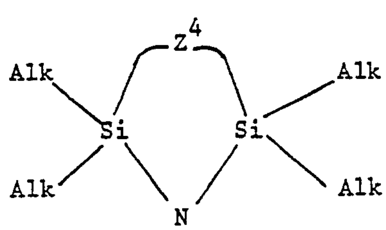

La transformation du composé II en organométallique correspondant (par exemple organolithien ou organomagnésien) est effectuée selon les méthodes usuelles.

Les dérivés organométalliques de formule II sont par exemple ceux pour lesquels $Z^2$ représente un métal alcalin ou un groupement —MgX, X étant un halogène.

Dans des modes d'exécution particuliers, le procédé de la présente demande peut encore présenter les caractéristiques suivantes :

l'agent d'acylation est une amine N-acylée, l'acyle étant dérivé d'un acide alkanoïque inférieur, ou un anhydride d'acide alcanoïque inférieur ; par exemple on peut utiliser comme agent de formylation la N-formyl pipéridine (G.A. Olah et M. Arvanaghi, Angew. Chem. Int. Ed. 1981, 878) ;

l'agent d'alkoxy-carbonylation est un chloroformiate d'alkyle inférieur ; on obtient dans ce cas un composé de formule III avec $Z^3$ représentant un groupement carbonyle et $R^4$ représentant un alkoxy inférieur. Autrement dit, le composé III est alors un ester que l'on peut transformer en amide correspondant selon les méthodes usuelles en passant éventuellement par l'acide carboxylique correspondant ou un de ses sels (sel métallique ou sel d'ammonium) ; par exemple on transforme ledit ester en amide par l'action de l'ammoniaque ;

l'agent d'acylation est un orthoester de formule :

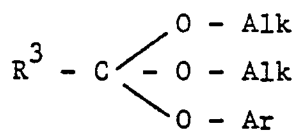

dans laquelle $R^3$ est un groupement —H ou un alkyle inférieur, —Alk est un alkyle inférieur et Ar est un groupement aromatique tel qu'un groupement phényle. On obtient alors un composé de formule III correspondant, avec $—Z^3—$ représentant un groupement cétal :

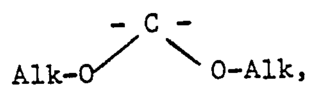

Dans ce cas, après la réaction de déprotection de l'amine on transforme $—Z^3—$, si désiré, en groupement carbonyle par hydrolyse du cétal selon méthodes connues.

Les composés de formule I peuvent être transformés en tout autre dérivé fonctionnel de la fonction carbonyle correspondant et/ou en tout sel d'addition correspondant par l'action d'un acide, par exemple un acide halohydrique.

L'invention a également pour objet, à titre de produits intermédiaires obtenus lors de la mise en œuvre du procédé décrit ci-dessus, les composés de formule VI :

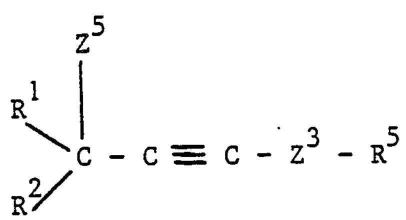

(VI)

dans laquelle soit $Z^5$ représente un groupement amine protégé, et dans ce cas ou bien $Z^3$ représente un groupement carbonyle ou un groupement précurseur du groupement carbonyle et $R^5$ représente —H ou un alkyle inférieur, ou bien $Z^3$ représente —CO— et $R^5$ est un alkoxy inférieur, soit $R^5$ représente un groupement $—NH_2$ et dans ce cas ou bien $Z^3$ représente un groupement carbonyle et $R^5$ représente un groupement $—OR^6$, $R^6$ étant —H, alkyle inférieur, un groupement ammonium ou un équivalent d'un métal, ou bien $Z^3$ représente un groupement carbonyle protégé et $R^5$ représente —H ou alkyle inférieur.

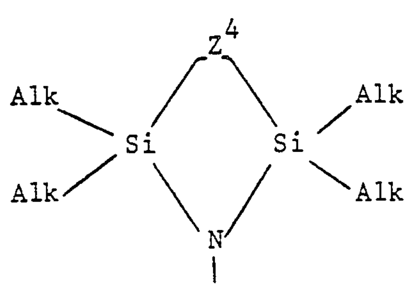

ceux pour lesquels ledit groupement précurseur du carbonyle est un groupement :

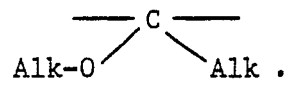

Les composés de formule I ainsi que leurs sels d'addition présentent des propriétés pharmacologiques intéressantes et notamment une propriété d'inhibition de l'activité aldéhyde déshydrogénase.

Ces propriétés, jointes à une toxicité réduite, permettent de les employer notamment dans le traitement de l'alcoolisme chronique. On sait en effet que les inhibiteurs d'aldéhyde déshydrogénase peuvent être utilisés dans les traitements de l'alcoolisme par désaccoutumance car ils empêchent la transformation de l'acétaldéhyde, métabolite de l'éthanol, en acide acétique, de sorte que l'acétaldéhyde est stocké dans le foie et provoque des réactions physiques passagères désagréables (nausées après absorption de boissons alcoolisées). Il en résulte progressivement une réaction de dégoût vis à vis des boissons alcoolisées ; voir par exemple Biochem. Pharmacology, 1982, Vol 31, 3613-3619.

D'autre part, on a découvert que les inhibiteurs d'aldéhyde déshydrogénase sont capables d'agir dans les cellules eucaryotes pour empêcher notamment la transformation du malonaldéhyde (obtenu sous l'action de la diamine oxydase) en acide malonique. L'accumulation du malonaldéhyde dans la cellule permet de bloquer la division cellulaire, l'activité du malonaldéhyde comme inhibiteur de la transcription et de la réplication ayant été démontrée in vitro.

On a également découvert que parmi les composés de formule I et leurs dérivés fonctionnels et/ou leurs sels d'addition, certains, y compris le composé de formule I pour lequel $R^1 = R^2 = R^3 = CH_3$, sont capables d'agir sélectivement sur les cellules transformées, probablement pour la raison qu'ils passent plus facilement à travers la paroi des cellules transformées qu'à travers la paroi des cellules normales.

Parmi les composés de formule I qui sont capables d'agir sélectivement sur les cellules transformées, on citera notamment les composés de formule I A :

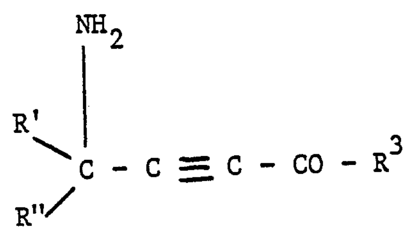

(IA)

dans laquelle R' et R" représentent indépendamment un groupement méthyle ou éthyle, et $R^3$ représente —H, alkyle inférieur ou —$NH_2$, ainsi que les cétals correspondants et les sels d'addition des composés de formule I A.

Parmi lesdits dérivés cétals, on citera en particulier ceux de formule I'A

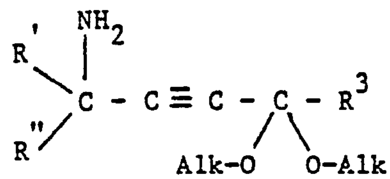

où R', R", et Alk sont définis comme précédemment, et $R^3$ représente —H ou alkyle inférieur.

Les composés de formule I A peuvent en conséquence être utilisés comme inhibiteurs sélectifs de la croissance des cellules transformées et peuvent trouver leur application en thérapeutique notamment dans le traitement des cancers comme agents anti-tumoraux en permettant notamment la régression des tumeurs et en empêchant l'établissement des métastases.

L'invention a également pour objet l'utilisation des composés de formule I ou I A, ainsi que de leurs cétals et/ou sels d'addition, comme médicaments.

A cet effet, ils sont présentés sous forme de composition pharmaceutique comprenant comme ingrédient actif au moins un composé de formule I ou I A, ou un sel d'addition correspondant.

Ces compositions pharmaceutiques comprennent en outre un excipient permettant l'administration du médicament par voie orale, intratumorale, intramusculaire, intraveineuse, ou sous forme d'implant.

La posologie varie avec la maladie traitée, le mode d'administration et l'état du malade. Généralement, la dose quotidienne de composé de formule I varie de 1 à 30 mg/kg de poids corporel, et le plus souvent de 5 à 25 mg/kg.

Les exemples suivants illustrent l'invention sans toutefois la limiter.

Dans ces exemples, les produits sont désignés par les numéros de formule suivants :

2a) 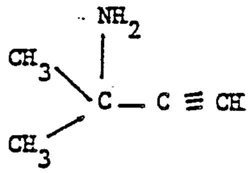

2b) 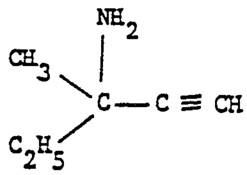

2c) 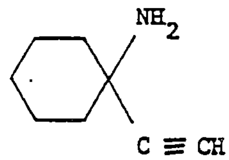

3a) 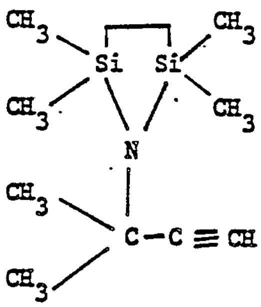

3b) 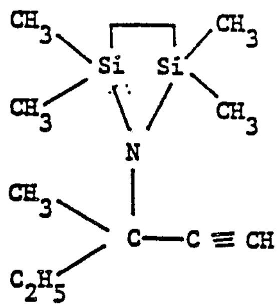

3c) 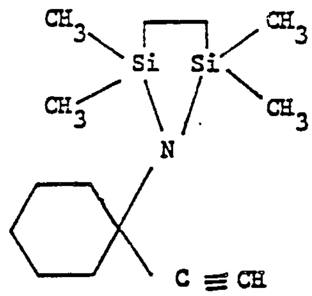

4a) 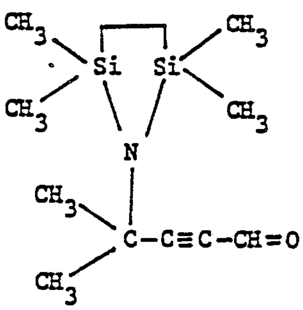

4b) 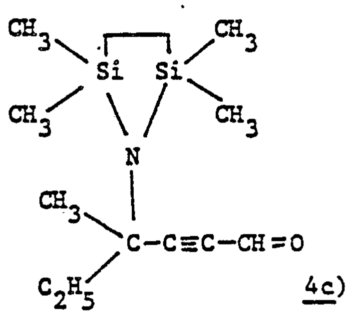

4c) 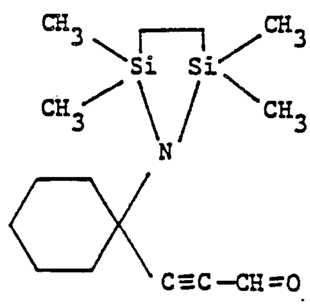

5a) 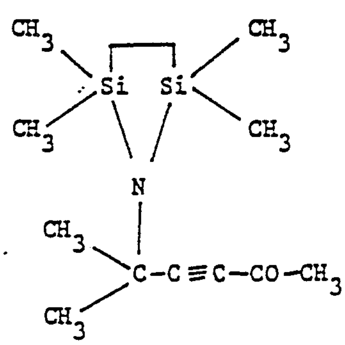

5b) 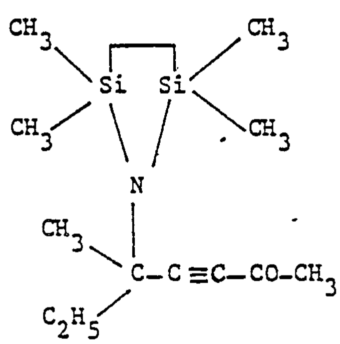

(Suite)

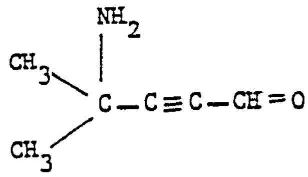

6a)

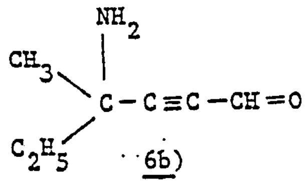

6b)

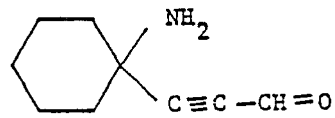

6c)

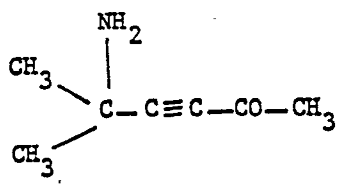

7a)

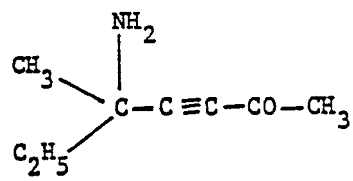

7b)

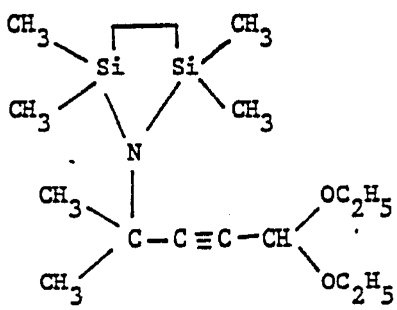

8a)

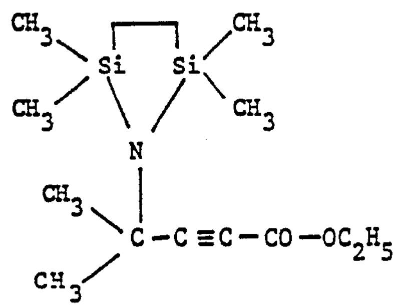

10a)

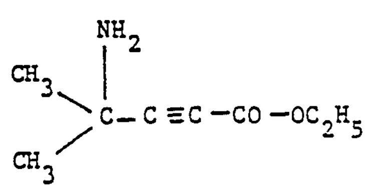

11a)

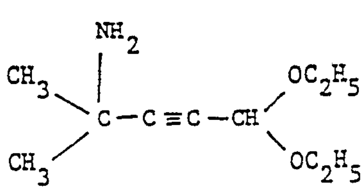

9a)

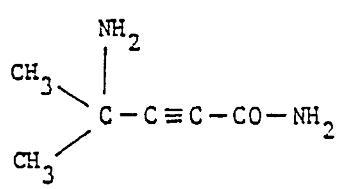

12a)

Exemple 1 — Préparation du composé 6a

Stade 1 : Protection du groupement aminé. Passage du composé 2a au composé 3a.

A une solution de 0,1 mole de l'amine 2a dans 100 ml de chlorure de méthylène anhydre, on additionne 0,25 mole de triéthylamine anhydre. On refroidit le milieu réactionnel à 0 °C puis ajoute, sous courant d'azote et agitation mécanique, goutte à goutte, une solution de 22,6 g (0,105 mole) de l'agent de protection du groupement amine, de formule :

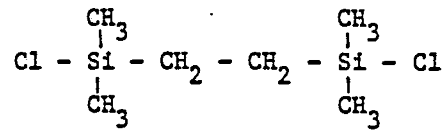

On agite à 0 °C pendant une heure puis on laisse revenir à température ambiante, après filtration du précipité de chlorhydrate de triéthylammonium et rinçage à l'éther éthylique, l'amine protégée 3a est obtenue sous forme de produit brut après évaporation du solvant. Elle est purifiée par distillation sous vide (Rdt = 80 %).
Eb : 45 °C/0,01 mm Hg. L'étude des spectres IR et de RMN $^1H$ confirme la structure indiquée.

Stade 2 : Formylation. Passage du composé 3a au composé 4a.

A une solution de 0,07 mole du composé 3a dans 300 ml d'éther éthylique anhydre refroidie à — 60 °C on ajoute, sous courant d'azote, 50 ml d'une solution de n-butyl lithium à 1,55 mole/litre (soit 0,075 mole) dans l'hexane.

On laisse remonter la température à — 30 °C en 30 minutes puis on coule une solution de 9 g (0,08 mole) de N-formyl pipéridine dans 10 ml d'éther éthylique anhydre. On laisse remonter lentement la température à 20 °C puis hydrolyse rapidement par une solution saturée de chlorure d'ammonium. Après lavage à l'eau, séchage et évaporation du solvant, on obtient le composé 4a que l'on utilise tel quel pour le stade suivant. Ce composé 4a peut être purifié par distillation. Eb : 78-80 °C/0,01 mm Hg.

L'étude des spectres IR et de RMN $^1H$ et $^{13}C$ confirme la structure indiquée.

Stade 3 : Déprotection du groupement amine. Passage du composé 4a au composé 6a (sous forme de chlorhydrate).

A une solution de 0,005 mole du composé 4a dans 20 ml d'éther éthylique refroidi à 0 °C on additionne goutte à goutte 30 ml d'une solution aqueuse d'acide chlorhydrique 0,2 N (1,2 éq).

Après 1 heure d'agitation à 0 °C, la phase aqueuse est décantée et réextraite deux fois par 20 ml d'éther éthylique puis stocké à — 20 °C.

La solution aqueuse de composé 6a (forme chlorhydrate) est tamponnée à pH 6,5 puis diluée par du sérum physiologique à la concentration voulue avant d'être testée.

Il convient de préciser que les solutions concentrées du produit 6a sont instables. Le produit est conservé sous forme de solutions diluées, par exemple le chlorhydrate peut être conservé en solution aqueuse diluée.

La structure du composé 6a a été confirmée par sa préparation au départ d'un autre précurseur, l'aminoacétal 9a (voir exemple 2).

De préférence, le produit 6a est donc conservé sous la forme du précurseur 9a qui est hydrolysé en produit 6a au moment de l'emploi.

Exemple 2 — Autre préparation du composé 6a

Stade 1 : Passage du composé 3a au composé 8a.

A une solution de 0,01 mole de composé 3a dans 40 ml de tétrahydrofuranne anhydre on additionne, sous courant d'azote, 20 ml d'une solution molaire de bromure d'éthyl-magnésium dans le tétrahydrofuranne (0,02 mole). Après la fin de l'addition, on porte au reflux du solvant jusqu'à ce que le dégagement d'éthane cesse, soit au bout d'une heure environ. On refroidit alors à 20 °C puis coule goutte à goutte une solution de 4,3 g (0,022 mole) d'orthoformiate de diéthyle et de phényle, de formule :

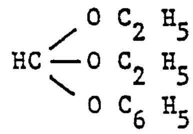

Après agitation à 20 °C pendant 16 heures, on hydrolyse par une solution saturée de chlorure d'ammonium. La phase organique est ensuite lavée par 3 fois 20 ml d'une solution de soude 5 N puis à l'eau jusqu'à neutralité. On obtient une solution de composé 8a que l'on utilise telle quelle au stade suivant. L'étude des spectres IR et de RMN $^1H$ confirme la structure indiquée.

Stade 2 : Passage du composé 8a au composé 9a.

On ajoute à la phase organique obtenue au stade précédent, 60 g de silice puis on chasse le solvant à l'évaporateur rotatif. La silice imprégnée par le mélange réactionnel est déposée sur une colonne de 50 g de silice puis on élue par un mélange méthanol-éther éthylique (20/80). On élue en tête l'excès d'orthoformiate de diéthyle et de phényle puis l'amino acétal pur 9a.

L'étude des spectres IR et de RMN $^1H$ ainsi que du spectre de masse confirme la structure indiquée.

Stade 3 : Passage du composé 9a au composé 6a (sous forme de chlorhydrate).

L'aminoacétal 9a est traité par 2 équivalents d'acide chlorhydrique 0,2 N pendant 18 heures à 27 °C. La solution aqueuse ainsi obtenue est amenée à pH 6,5 par la soude 1 N, puis diluée par du sérum physiologique à la concentration voulue.

Les solutions du produit obtenu présentent les mêmes caractéristiques que celles obtenues avec le produit préparé selon l'exemple 1, notamment en ce qui concerne le dosage à 620 nm de la fonction aldéhyde à l'aide du dérivé préparé par réaction de la 3-méthyl benzo-2 thiazolinone hydrazone (MBTH) en présence de sels ferriques.

De plus, les solutions de 6a (chlorhydrate) obtenues par cette seconde méthode ont une activité en tous points comparable à celle observée avec le produit de l'exemple 1 concernant l'inhibition de la croissance en culture de tissus de cellules NRK (normales) et NRK-B77 (transformées).

## Exemple 3 — Préparation du composé 6b

Stade 1 : Passage du composé 2b au composé 3b.

On opère de façon analogue à celle décrite à l'exemple 1, stade 1. On obtient le composé 3b. Eb : 70 °C/0,01 mm Hg. L'étude des spectres IR et de RMN $^1H$ confirme la structure indiquée.

Stade 2 : Passage du composé 3b au composé 4b.

On opère de façon analogue à celle décrite à l'exemple 1, stade 2. L'étude du spectre IR et de RMN $^1H$ confirme la structure indiquée pour 4b.

Stade 3 : Passage du composé 4b au composé 6b.

On opère de façon analogue à celle décrite à l'exemple 1, stade 3.

## Exemple 4 — Préparation du composé 6c

Stade 1 : Passage du composé 2c au composé 3c.

On opère de façon analogue à celle décrite à l'exemple 1, stade 1. On obtient le composé 3c ; Eb : 80 °C/0,01 mm Hg. L'étude des spectres IR et de RMN $^1H$ confirme la structure indiquée.

Stade 2 : Passage du composé 3c au composé 4c.

On opère de façon analogue à celle décrite à l'exemple 1, stade 3. L'étude des spectres IR et de RMN $^1H$ confirme la structure indiquée pour 4c.

Stade 3 : Passage du composé 4c au composé 6c.

On opère de façon analogue à celle décrite à l'exemple 1, stade 3.

Exemple 5 — Préparation du composé 7a

Stade 1 : Acétylation. Passage du composé 3a au composé 5a.

A une solution de 0,016 mole de composé 3a dans 100 ml d'éther éthylique anhydre refroidie à — 60 °C, on additionne, sous courant d'azote, 11,4 ml d'une solution à 1,55 mole/litre de n-butyl lithium (soit 0,0176 mole) dans l'hexane. On laisse remonter la température à — 10 °C en 30 minutes puis refroidit à nouveau à — 50 °C et la solution est introduite dans une ampoule de coulée isobare réfrigérée à — 20 °C, puis additionné goutte à goutte, sous courant d'azote, à une solution de 5 g (0,048 mole) d'anhydride acétique dans 30 ml de tétrahydrofuranne anhydre maintenu à — 15 °C. Après agitation pendant 1 heure 30 minutes à — 15 °C, on additionne à une solution de 4 g de bicarbonate de sodium dans 50 ml d'eau. Quand le dégagement de gaz carbonique a cessé, on décante et extrait la phase aqueuse à l'éther éthylique. Après lavage à l'eau de la phase organique jusqu'à neutralité, séchage et évaporation du solvant, on obtient le composé 5a que l'on peut purifier par distillation. Eb : 80-82 °C/0,01 mm Hg. L'étude des spectres IR et de RMN $^1H$ confirme la structure indiquée.

Stade 2 : Passage du composé 5a au composé 7a.

On opère de façon analogue à celle décrite à l'exemple 1, stade 3.

Exemple 6 — Préparation du composé 7b

Stade 1 : Passage du composé 3b au composé 5b.

On opère de façon analogue à celle mentionnée à l'exemple 5, stade 1. On obtient le composé 5b. L'étude des spectres IR et de RMN $^1H$ confirme la structure indiquée.

Stade 2 : Passage du composé 5b au composé 7b.

On opère de façon analogue à celle décrite à l'exemple 5, stade 2.

Exemple 7 — Préparation du composé 12a

Stade 1 : Ethoxycarbonylation. Passage du composé 3a au composé 10a.

A une solution de 0,01 mole du dérivé lithié du composé 3a dans 70 ml de tétrahydrofuranne, préparé comme décrit précédemment à l'exemple 1, stade 2, on additionne à — 60 °C, goutte à goutte, sous courant d'azote, une solution de 1,4 g (0,011 mole) de chloroformiate d'éthyle dans 15 ml de tétrahydrofuranne. On laisse la température remonter lentement (au bout de deux heures environ) à 0 °C puis hydrolyse par une solution saturée de chlorure d'ammonium. Après extraction à l'éther, lavage et évaporation du solvant on obtient le composé 10a utilisé tel quel pour l'étape suivante.

Stade 2 : Déprotection du groupement amine. Passage du composé 10a au composé 11a.

On opère de façon analogue à celle décrite à l'exemple 1, stade 3. La fonction amine est déprotégée par traitement à l'acide chlorhydrique 1 N (1,1 éq) pendant 30 mn puis neutralisation à la soude 1 N. L'amino ester 11a est purifié par chromatographie sur colonne de silice (éluant : acétate d'éthyle). Les spectres IR, de RMN $^1H$ et de masse confirment la structure indiquée.

Stade 3 : Amidification. Passage du composé 11a au composé 12a.

On traite 0,230 g d'amino ester 11a obtenu au stade précédent, en solution dans 0,3 ml de méthanol, par 0,3 ml d'ammoniaque dans un flacon bouché pendant 16 heures. Après extraction à l'éther éthylique, l'aminoamide obtenu 12a est purifié sur colonne de silice (éluant : méthanol). Le spectre IR et les spectres de RMN $^1H$ et de masse confirment la structure indiquée.

Etude des propriétés pharmacologiques

1. Résultats des tests « in vitro »

1.1. Inhibition de l'activité aldéhyde déshydrogénase

L'activité d'aldéhyde déshydrogénase a été recherchée par le changement de l'absorption du cofacteur NADP transformé en NADPH, à 340 nm, en présence de benzaldéhyde comme substrat. La pré-incubation de l'enzyme avec le produit étudié (6a, sous forme de chlorhydrate) amène une diminution de 64 % de la densité optique due à la transformation NADP — NADPH.

1.2. Inhibition de la croissance de cellules transformées

Les tableaux 1 et 2 ci-après donnent le résultat de ces tests qui portent sur trois types de cellules transformées. Les chiffres indiqués représentent le pourcentage d'inhibition de la croissance de cellules traitées par des doses de plus en plus fortes de composé 6a (chlorhydrate) par rapport à celles des mêmes cellules non traitées.

On peut constater que le produit étudié est un inhibiteur sélectif de croissance des cellules transformées, qu'elles soient d'origine animale ou humaine, et que l'inhibition de la croissance des cellules normales est nulle ou très réduite.

Le produit est efficace contre la transformation des cellules d'embryon de poulet induite par le virus du sarcome de ROUS.

Il faut noter que la réplication du virus n'est pas inhibée contrairement à ce qui est observé avec les agents anti-tumoraux connus.

2. Tests de Toxicité

Les tests dont les résultats sont donnés ci-dessous ont été réalisés avec des solutions du composé 6a (sous forme de chlorhydrate). Dans ces tests, on a étudié l'activité vis à vis de la croissance tumorale (carcinome et mélanome) et la survie de souris swiss auxquelles ont été injectées des quantités variables de cellules d'ascites de Krebs.

Toxicité aigüe : La DL50 du composé 6a (chlorhydrate) déterminée sur ces souris par deux mesures indépendantes est de 60 mg/kg.

Toxicité chronique : De plus, le composé 6a (chlorhydrate) n'a montré aucune toxicité :

vis-à-vis de cellules de la membrane amniotique humaine (aucun effet sur la croissance et sur la morphologie des cellules n'a été observé pour des concentrations de $5 \cdot 10^{-5}$ et de $10^{-4}$ mole-litre $^{-1}$) ;

vis-à-vis du clone FAZA 967 de l'hépatome de rat REUBER H 35, (aucune toxicité du produit, estimée par dosage dans le milieu des enzymes lactate déshydrogénase (LDH), glutamic oxaloacetate transaminase (GOT) et glutamic pyruvate transaminase (GPT) n'a été constatée) ;

sur des souris suisses soumises à un traitement de 2 micromoles de produit étudié par jour pendant trois semaines.

3. Tests « in vivo »

3.1. Croissance cellulaire

Deux types de tumeurs ont été utilisés :

un carcinome de Lewis (sur souris swiss),

un mélanome DAU d'origine humaine (sur souris nude).

3.1.1. Mélanome

Les souris ont reçu une injection de $5 \cdot 10^5$ cellules de mélanome DAU. Celles traitées ont reçu :

$2 \times 15$ mg/kg le premier jour,

$2 \times 6$ mg/kg du deuxième au septième jour.

On observe chez 60 % des souris traitées un retard d'une dizaine de jours dans le développement tumoral, par rapport aux témoins non traités.

3.1.2. Carcinome

Le test est effectué sur des souris auxquelles $3 \cdot 10^5$ cellules de carcinome de Lewis ont été inoculées. On peut observer un net ralentissement de la croissance tumorale chez les souris traitées journellement par 6 mg/kg ou par 12 mg/kg du produit 6a (chlorhydrate).

3.2. Survie

Les résultats des tests ont été obtenus principalement à partir de souris auxquelles ont été injectées des cellules d'ascites de KREBS (carcinome) dont l'origine a été décrite par G. Klein et E. Klein Cancer Research 1951-11-466. Dans le cas d'une inoculation de $10^5$ cellules, une injection journalière de 6 mg/kg du chlorhydrate de 6a pendant 7 jours est déjà suffisante pour obtenir la guérison complète jusque 6 mois. Dans le cas où $5 \cdot 10^5$ cellules ont été inoculées, la durée du traitement nécessaire est allongée : un traitement de 7 jours à 6 mg/kg provoque un retard dans la mort mais n'augmente pas le taux de survie alors qu'un traitement de 21 jours accroît ce taux jusque 90 %.

Le même effet peut être obtenu si l'on traite d'entrée par des quantités plus importantes ; par exemple $2 \times 15$ mg/kg le 1er jour puis $2 \times 6$ mg/kg les 6 jours suivants.

Ces mêmes posologies et mode d'administration sont encore suffisants pour $10^6$ cellules mais leur efficacité est moindre dans le cas de l'inoculation de $5 \cdot 10^6$ cellules.

Une étude a aussi été faite sur des souris auxquelles avaient été inoculées $3 \cdot 10^5$ cellules de leucémie L1210. Le produit 6a (chlorhydrate) injecté à raison de 6 mg/kg pendant 7 jours a exercé le même effet que dans le cas du carcinome.

En conclusion, toutes ces études faites in vivo montrent une corrélation entre la quantité du produit étudié et le nombre de cellules transformées injectées dont il est possible d'empêcher le développement.

En outre, le composé 9a a été trouvé actif sélectivement in vivo contre certaines lignées de lymphomes de souris.

Tableau 1

Effet du produit 6a (Chlorhydrate) sur la croissance cellulaire (exprimé en % d'inhibition)

| Type de Cellules | Concentration finale de produit étudié | | | | |
|---|---|---|---|---|---|
| | $5.10^{-5}$M | $1.10^{-4}$M | $2.10^{-4}$M | $4.10^{-4}$M | $6.10^{-4}$M |
| Cellules normales | | | | | |
| Cellules d'embryon de poulet | 0 | 0 | 0 | 10 | 12 |
| Fibroblastes humains MRC 5 | 0 | 0 | 0 | 0 | 18 |
| Cellules de la membrane amniotique humaine | - | - | - | - | 0 |
| Cellules transformées | | | | | |
| Cellules de rein de rat NRK B 77 | 0 | 16 | 31 | 57 | 79 |
| NRK LA31 à 33° | 0 | 0 | 11 | 63 | Mort |
| Cellules HeLa humaines | 0 | 18 | 36 | 78 | 91 |
| Hepatomes de rat | - | - | 50 | - | 60 |

(Voir tableau page 13)

Tableau 2

Tests in vitro complémentaires effectués sur des cellules de mélanomes humains M3 DAU et M1 DOR, % de cellules mortes après quatre jours de croissance :

| | Concentration en produit étudié | | | | | |
|---|---|---|---|---|---|---|
| | 0 | $1,5.10^{-3}$ | $2.10^{-3}$ | $3.10^{-3}$ | $4.10^{-3}$ | $5.10^{-3}$ |
| M3 DAU (100 % de tumeurs chez la souris nude) | 6 | 24 | 47 | 92 | 98 | 98 |
| M1 DOR (25 % de tumeurs chez la souris nude) | 24 | 21 | 28 | 24 | 26 | 23 |

**Revendications**

1. Les composés de formule générale I :

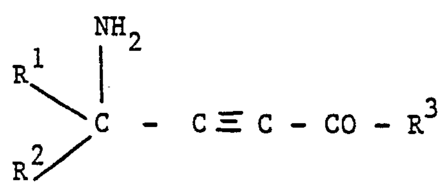

(I)

dans laquelle $R^1$ et $R^2$ représentent chacun indépendamment un groupement alkyle ayant de 1 à 5 atomes de carbone, ou bien $R^1$ et $R^2$ représentent ensemble un groupement alkylène ayant 4 ou 5 atomes de carbone, et $R^3$ représente —H, un groupement alkyle inférieur ou un groupement $—NH_2$ ; ainsi que les cétals correspondants et les sels d'addition des composés de formule I, et les solutions contenant lesdits composés ou sels d'addition ; à l'exception du composé de formule I pour lequel $R^1 = R^2 = CH_3$ et $R^3 = CH_3$.

2. Composés selon la revendication 1, caractérisés par le fait que $R^3$ représente un groupement alkyle ayant 1 à 5 atomes de carbone.

3. Composés selon l'une quelconque des revendications 1 et 2, caractérisés par le fait que $R^3$ est choisi parmi les groupements —H, $—CH_3$ et $—NH_2$.

4. Composés selon la revendication 1, caractérisés par le fait qu'ils sont choisis parmi les composés de formule I suivants :

$R^1 = R^2 = CH_3$ et $R^3 = H$ ;
$R^1 = R^2 = CH_3$ et $R_3 = —NH_2$ ;
$R^1 = CH_3$, $R^2 = C_2H_5$ et $R^3 = H$ ;
$R^1 = R^3 = CH_3$ et $R^2 = C_2H_5$ ;
$R^1 + R^2 = —(CH_2)_5—$ et $R^3 = H$ ; ainsi que les cétals et/ou sels d'addition de ces composés.

5. Composés selon l'une quelconque des revendications 1 et 2, caractérisés par le fait qu'ils répondent à la formule :

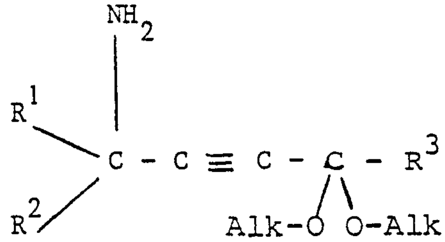

où Alk est un alkyle inférieur, et $R^1$ et $R^2$ sont définis comme précédemment, et $R^3$ représente —H ou un groupement alkyle inférieur.

6. Composés selon l'une quelconque des revendications précédentes, caractérisés par le fait qu'ils sont choisis parmi les sels d'addition que forment les composés de formule I avec les acides compatibles avec une utilisation en thérapeutique.

7. Procédé de préparation des composés de formule I tels que définis dans l'une quelconque des revendications précédentes, caractérisé par le fait que l'on utilise comme produit de départ un composé de formule II :

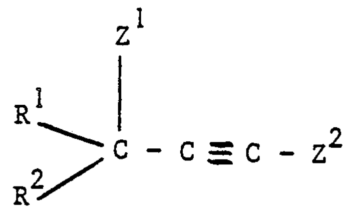

(II)

dans laquelle $R^1$ et $R^2$ sont définis comme précédemment, $Z^1$ représente un groupement amine protégé (la liaison C—$Z^1$ étant une liaison carbone-azote), et $Z^2$ représente soit —H soit un équivalent d'un métal ou d'un groupement lié à l'atome de carbone auquel il est attaché par une liaison carbone-métal, que l'on fait réagir ledit composé de départ avec un agent d'acylation ou d'alkoxycarbonylation, pour obtenir un composé de formule III :

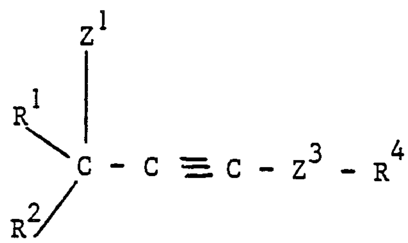

dans laquelle $R^1$, $R^2$ et $Z^1$ sont définis comme précédemment, $R^4$ représente —H, alkyle inférieur et $Z^3$ représente soit un groupement carbonyle —CO—, soit un groupement précurseur du groupement carbonyle, ou $R^4$ est un alkoxy inférieur et $Z^3$ est alors un groupement —CO— puis que l'on soumet le composé obtenu à une réaction de déprotection du groupement amine et que, le cas échéant, soit on transforme si désiré, le groupement —$Z^3$— en groupement carbonyle, soit, dans le cas où —$Z^3$—$R^4$ représente un groupement alkoxycarbonyle, on transforme l'ester obtenu en amide correspondant selon les méthodes usuelles en passant éventuellement par l'acide carboxylique ou un de ses sels métalliques ou le sel d'ammonium correspondant, et on obtient le composé de formule I désiré ou un sel d'addition correspondant que l'on transforme si désiré, en cétal et/ou en sel d'addition correspondant.

8. Procédé selon la revendication 7, caractérisé par le fait que $Z^2$ représente un atome d'hydrogène, un métal alcalin ou un groupement —MgX, X étant un halogène.

9. Procédé selon l'une quelconque des revendications 7 et 8, caractérisé par le fait que ledit agent d'acylation est une amine N-acylée (l'acyle étant dérivé d'un acide alcanoïque inférieur) ou un anhydride d'acide alcanoïque inférieur.

10. Procédé selon la revendication 8, caractérisé par le fait que ledit agent d'acylation est la N-formyl pipéridine.

11. Procédé selon l'une quelconque des revendications 7 et 8, caractérisé par le fait que ledit agent d'alkoxy-carbonylation est un chloroformiate d'alkyle inférieur.

12. Procédé selon la revendication 7, caractérisé par le fait que l'on transforme ledit ester en amide par l'action de l'ammoniaque.

13. Procédé selon l'une quelconque des revendications 7 et 8, caractérisé par le fait que ledit agent d'acylation est un orthoester de formule :

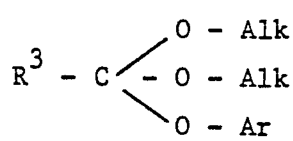

où $R^3$ est un groupement —H ou un alkyle inférieur, —Alk est un alkyle inférieur et Ar est un groupement aromatique tel qu'un groupement phényle, que l'on obtient un composé de formule III correspondant, avec —$Z^3$— représentant

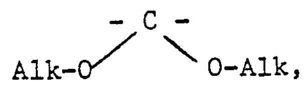

et qu'après la réaction de déprotection de l'amine on transforme —$Z^3$—, si désiré, en groupement carbonyle par hydrolyse du cétal selon les méthodes connues.

14. Procédé selon l'une quelconque des revendications 7 à 13, caractérisé par le fait que $Z^1$ représente un groupement :

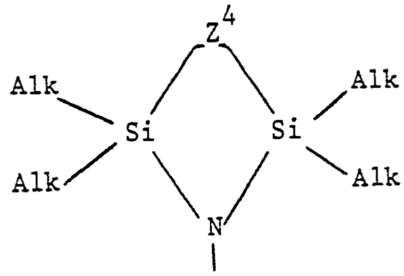

dans laquelle Alk est un alkyle inférieur et $Z^4$ est un groupement alkylène ayant 2 ou 3 atomes de carbone dans la chaîne, et que l'on effectue la réaction de déprotection de l'amine par hydrolyse acide.

15. Compositions pharmaceutiques, caractérisées par le fait qu'elles renferment comme ingrédient actif au moins un composé choisi parmi les composés de formule I

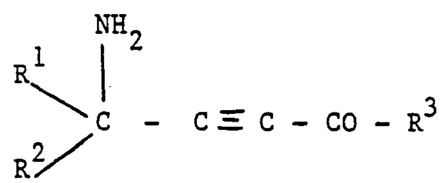

(I)

dans laquelle $R^1$ et $R^2$ représentent chacun indépendamment un groupement alkyle ayant 1 à 5 atomes de carbone, ou bien $R^1$ et $R^2$ représentent ensemble un groupement alkylène ayant 4 ou 5 atomes de carbone et $R^3$ représente —H, un groupement alkyle inférieur ou un groupement —$NH_2$ ; ainsi que les cétals correspondants et les sels d'addition des composés de formule I, et les solutions contenant lesdits composés ou sels d'addition.

16. Compositions pharmaceutiques selon la revendication 15, caractérisées par le fait qu'elles renferment comme ingrédient actif soit au moins un composé de formule I A :

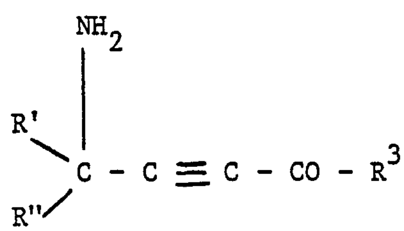

(IA)

dans laquelle R′ et R″ représentent indépendamment un groupement méthyle ou éthyle, et $R^3$ représente —H, alkyle inférieur ou —$NH_2$, soit au moins un cétal et/ou un sel d'addition d'un composé I A.

17. Compositions selon la revendication 15, caractérisées par le fait qu'elles renferment comme ingrédient actif au moins un composé de formule

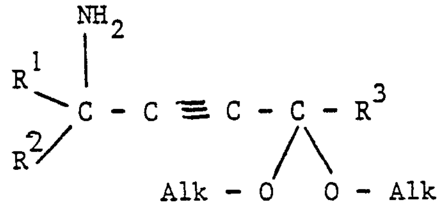

où Alk est un alkyle inférieur, $R^1$ et $R^2$ sont définis comme précédemment, et $R^3$ représente —H ou un

groupement alkyle inférieur.

18. A titre de produits intermédiaires obtenus dans le procédé de l'une des revendications 7 à 14, les composés de formule VI :

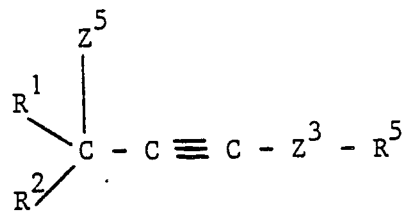

(VI)

dans laquelle soit $Z^5$ représente un groupement amine protégé, et dans ce cas ou bien $Z^3$ représente un groupement carbonyle ou un groupement précurseur du groupement carbonyle et $R^5$ représente —H ou un alkyle inférieur, ou bien $Z^3$ représente —CO— et $R^5$ est un alkoxy inférieur, soit $Z^5$ représente un groupement $—NH_2$ et dans ce cas ou bien $Z^3$ représente un groupement carbonyle et $R^5$ représente un groupement $—OR^6$, $R^6$ étant —H, alkyle inférieur, un groupement ammonium ou un équivalent d'un métal, ou bien $Z^3$ représente un groupement carbonyle protégé et $R^5$ représente —H ou alkyle inférieur.

19. A titre de produits intermédiaires selon la revendication 18, les composés de formule VI pour lesquels $Z^5$ représente :

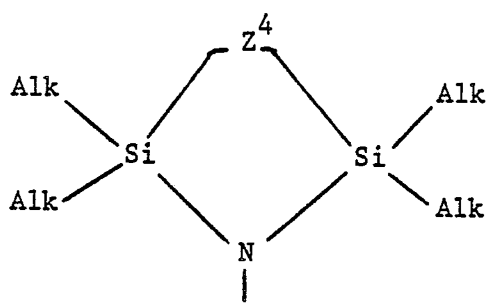

20. A titre de produits intermédiaires selon la revendication 18 ou 19, les composés de formule VI pour lesquels ledit groupement précurseur du carbonyle est un groupement :

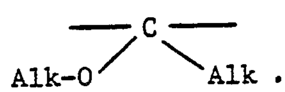

**Claims**

1. The compounds of the general formula I

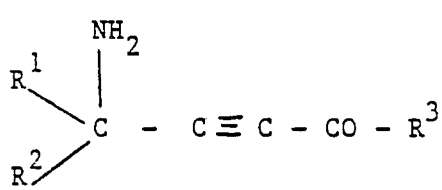

(I)

in which $R^1$ and $R^2$ each independently represent an alkyl group having from 1 to 5 carbon atoms, or $R^1$ and $R^2$ together represent an alkylene group having 4 or 5 carbon atoms, and $R^3$ represents —H, a lower alkyl group or a $—NH_2$ group, as well as the corresponding ketals and the addition salts of the compounds of formula I, and the solutions containing the said compounds or addition salts, with the exception of the compound of formula I in which $R^1 = R^2 = CH_3$ and $R^3 = CH_3$.

2. Compounds according to Claim 1, characterized in that $R^3$ represents an alkyl group having 1 to 5 carbon atoms.

3. Compounds according to any one of Claims 1 and 2, characterized in that $R^3$ is chosen from among the —H, $—CH_3$ and $—NH_2$ groups.

4. Compounds according to Claim 1, characterized in that they are chosen from among the following compounds of formula 1 :

$R^1 = R^2 = CH_3$ and $R^3 = H$ ;

$R^1 = R^2 = CH_3$ and $R_3 = -NH_2$ ;
$R^1 = CH_3$, $R^2 = C_2H_5$ and $R^3 = H$ ;
$R^1 = R^3 = CH_3$ and $R^2 = C_2H_5$ ;
$R^1 + R^2 = -(CH_2)_5-$ and $R^3 = H$, as well as the ketals and/or addition salts of these compounds.

5. Compounds according to either of Claims 1 and 2, characterized in that they correspond to the formula :

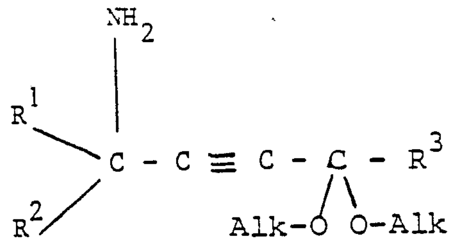

where Alk is a lower alkyl and $R^1$ and $R^2$ are defined as above, and $R^3$ represents —H or a lower alkyl group.

6. Compounds according to any one of the preceding claims, characterized in that they are chosen from among the addition salts which the compounds of formula I form with acids which are compatible with therapeutic use.

7. Process of preparation of the compounds of formula I as defined in any one of the preceding claims, characterized in that the starting product used is a compound of formula II :

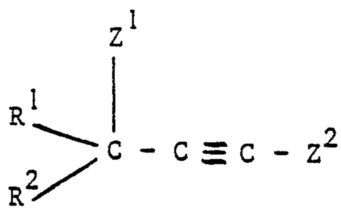

(II)

in which $R^1$ and $R^2$ are defined as above, $Z^1$ represents a protected amine group (the $C-Z^1$ bond being a carbonnitrogen bond) and $Z^2$ represents either —H or one equivalent of a metal or of a group bonded to the carbon atom to which it is attached by a carbon-metal bond, that the said starting compound is reacted with an acylating agent or alkoxycarbonylating agent, to give a compound of formula III :

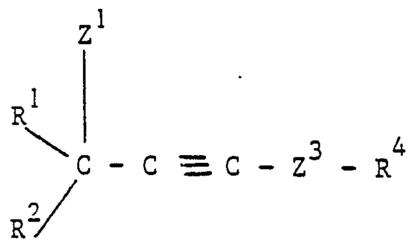

in which $R^1$, $R^2$ and $Z^1$ are defined as above, $R^4$ represents —H or lower alkyl and $Z^3$ represents either a carbonyl group —CO—, or a precursor group of the carbonyl group, or $R^4$ is a lower alkoxy and $Z^3$ is in that case a —CO— group, after which the compound obtained is subjected to a deprotection reaction of the amine group and that, where appropriate, either, if desired, the $-Z^3-$ group is converted to a carbonyl group or, in the case where $-Z^3-R^4$ represents an alkoxycarbonyl group, the ester obtained is converted to the corresponding amide in accordance with the usual methods, where appropriate via the carboxylic acid or one of its metal salts or the corresponding ammonium salt, and the desired compound of formula I or a corresponding addition salt is obtained and is converted, if desired, to a corresponding addition salt and/or ketal.

8. Process according to Claim 7, characterized in that $Z^2$ represents a hydrogen atom, an alkali metal or a —MgX group, X being a halogen.

9. Process according to either of Claims 7 and 8, characterized in that the said acylating agent is a N-acylated amine (the acyl being derived from a lower alkanoic acid) or an anhydride of a lower alkanoic acid.

10. Process according to Claim 8, characterized in that the said acylating agent is N-formylpiperidine.

11. Process according to either of Claims 7 and 8, characterized in that the said alkoxycarbonylating agent is a lower alkyl chloroformate.

12. Process according to Claim 7, characterized in that the said ester is converted to an amide by the action of ammonia.

13. Process according to either of Claims 7 and 8, characterized in that the said acylating agent is an ortho-ester of the formula :

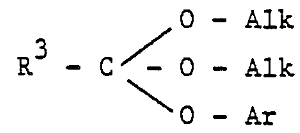

where $R^3$ is a —H or lower alkyl group, —Alk is a lower alkyl and Ar is an aromatic group such as a phenyl group, that a corresponding compound of formula III, with —$Z^3$— representing

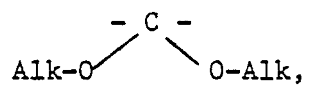

is obtained and that after the amine deprotection reaction —$Z^3$— is converted, if desired, to a carbonyl group by hydrolysis of the ketal in accordance with known methods.

14. Process according to any one of Claims 7 to 13, characterized in that $Z^1$ represents a

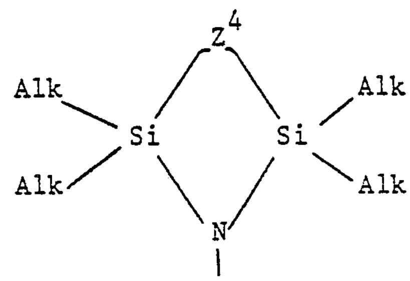

group, in which Alk is a lower alkyl and $Z^4$ is an alkylene group having 2 or 3 carbon atoms in the chain, and that the amine deprotection reaction is carried out by acid hydrolysis.

15. Pharmaceutical compositions, characterized in that they contain, as the active ingredient, at least one compound chosen from among the compounds of formula I

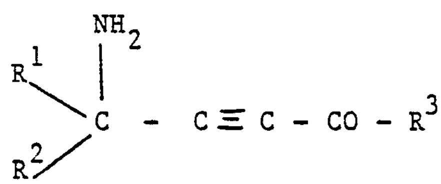

(I)

in which $R^1$ and $R^2$ each independently represent an alkyl group having from 1 to 5 carbon atoms, or $R^1$ and $R^2$ together represent an alkylene group having 4 or 5 carbon atoms, and $R^3$ represents —H, a lower alkyl group or a —$NH_2$ group, as well as the corresponding ketals and the addition salts of the compounds of formula I, and the solutions containing the said compounds or addition salts.

16. Pharmaceutical compositions according to Claim 15, characterized in that they contain, as the active ingredient, either at least one compound of formula I A :

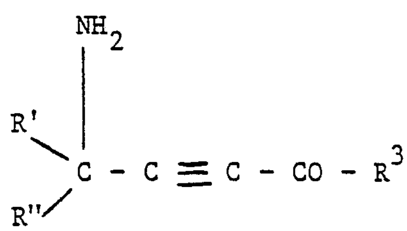

(IA)

in which R′ and R″ independantly represent a methyl or ethyl group and $R^3$ represents —H, lower alkyl or —$NH_2$, or at least one ketal and/or one addition salt of a compound I A.

17. Compositions according to Claim 15, characterized in that they contain, as the active ingredient, at least one compound of formula

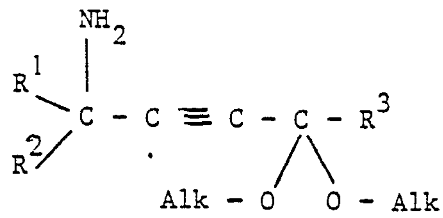

where Alk is a lower alkyl, $R^1$ and $R^2$ are defined as above and $R^3$ represents —H or a lower alkyl group.

18. By way of intermediate products obtained in the process of one of Claims 7 to 14, the compounds of formula VI :

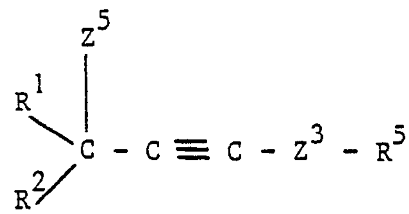

(VI)

in which either $Z^5$ represents a protected amine group in which case either $Z^3$ represents a carbonyl group or a precursor group of the carbonyl group and $R^5$ represents —H or lower alkyl or $Z^3$ represents —CO— and $R^5$ is a lower alkoxy, or $Z^5$ represents a $—NH_2$ group, in which case either $Z^3$ represents a carbonyl group and $R^5$ represents a $—OR^6$ group, $R^6$ being —H, lower alkyl, an ammonium group or one equivalent of a metal, or $Z^3$ represents a protected carbonyl group and $R^5$ represents —H or lower alkyl.

19. By way of intermediate products according to Claim 18, the compounds of formula VI for which $Z^5$ represents :

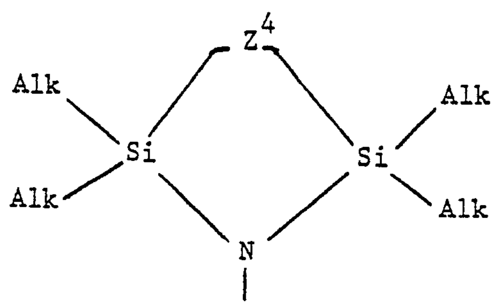

20. By way of intermediate products according to Claim 18 or 19, the compounds of formula VI for which the said precursor group of the carbonyl group is a :

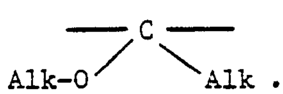

group.

**Patentansprüche**

1. Verbindungen der allgemeinen Formel I :

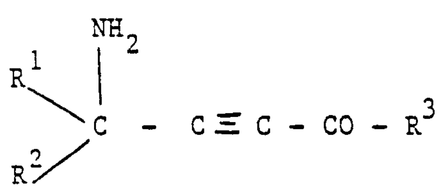

(I)

in der $R^1$ und $R^2$ jeweils unabhängig voneinander eine Alkylgruppe von 1 bis 5 Kohlenstoffatomen bedeuten oder $R^1$ und $R^2$ zusammen eine Alkylengruppe mit 4 oder 5 Kohlenstoffatomen darstellen und $R^3$—H, eine niedere Alkylgruppe oder eine Gruppe $NH_2$ bedeutet sowie die entsprechenden Ketale und die Additionssalze der Verbindungen der Formel I und Lösungen, die diese Zubereitungen oder Additionssalze enthalten, ausgenommen einer Verbindung der Formel I, in der $R^1 = R^2 = CH_3$ und $R^3 = CH_3$ ist.

2. Verbindungen nach Anspruch 1, dadurch gekennzeichnet daß $R^3$ eine Alkylgruppe von 1-5 Kohlenstoffatomen bedeutet.

3. Verbindungen nach jedem der Ansprüche 1 und 2, dadurch gekennzeichnet, daß $R^3$ ausgewählt ist aus den Gruppen —H, —$CH_3$ und —$NH_2$.

4. Verbindungen nach Anspruch 1, dadurch gekennzeichnet daß sie aus folgenden Verbindungen der Formel I ausgewählt sind :

$R^1 = R^2 = CH_3$, $R^3 = H$ ;
$R^1 = R^2 = CH_3$, $R_3 = —NH_2$ ;
$R^1 = CH_3$, $R^2 = C_2H_5$, $R^3 = H$ ;
$R^1 = R^3 = CH_3$, $R^3 = C_2H_5$ ;
$R^1 + R^2 = —(CH_2)_5—$, $R^3 = H$ ;

sowie die Ketale und/oder Additionssalze dieser Verbindungen.

5. Verbindungen nach einem der Ansprüche 1 und 2, dadurch gekennzeichnet, daß sie der folgenden Formel entsprechen :

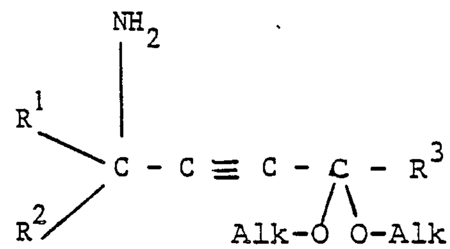

wobei Alk eine niedere Alkylgruppe ist und $R^1$ und $R^2$ die oben genannte Bedeutung haben und $R^3$ —H oder eine niedere Alkylgruppe bedeutet.

6. Verbindungen nach jedem der vorhergehenden Ansprüche, dadurch gekennzeichnet, daß sie ausgewählt sind aus den Additionssalzen, die von Verbindungen der Formel I mit verträglichen Säuren mit therapeutischer Anwendungsmöglichkeit gebildet sind.

7. Verfahren zur Herstellung von Verbindungen der Formel I, wie sie in den vorher genannten Ansprüchen definiert sind, dadurch gekennzeichnet, daß man als Ausgangsmaterial eine Verbindung der Formel II verwendet

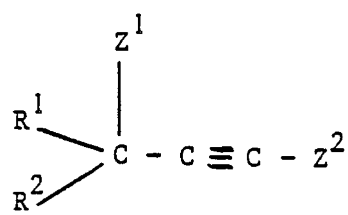

(II)

in der $R^1$ und $R^2$ wie oben definiert sind und $Z^1$ eine geschützte Amingruppe darstellt (wobei die Bindung C—$Z^1$ eine Kohlenstoff-Stickstoff-Bindung ist) und $Z^2$ entweder —H oder das Äquivalent eines Metalls oder eine Gruppe, die mit einem Kohlenstoffatom, welches an eine Kohlenstoff-Metall-Bindung gebunden ist, darstellt, daß man diese Ausgangsverbindung mit einem Acylierungsmittel oder einem Alkoxycarbonylierungsmittel zu einer Verbindung der folgenden Formel III umsetzt :

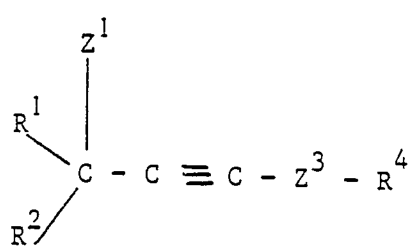

worin $R^1$, $R^2$ und $Z^1$ die oben genannte Bedeutung haben, $R^4$ —H, oder eine niedere Alkylgruppe bedeuten und $Z^3$ entweder eine Carbonylgruppe —CO— oder eine Carbonylvorläufergruppe bedeutet oder $R^4$ eine niedere Alkoxygruppe und $Z^3$ ebenfalls eine Gruppe —CO— darstellt und daß man die erhaltene Verbindung einer Reaktion unterwirft, bei der die Amin-Schutzgruppe abgespalten wird und daß man gegebenenfalls entweder die Gruppe —$Z^3$— in eine Carbonylgruppe überführt oder gewünschtenfalls im Fall daß —$Z^3$—$R^4$ eine Alkoxycarbonylgruppe ist, den erhaltenen Ester in das entsprechende Amid mit an sich bekannten Methoden überführt und gegebenenfalls diese Verbindung in die entsprechende Karbonsäure oder deren Metallsalz überführt oder in das entsprechende Ammoniumsalz überführt und die erhaltene gewünschte Verbindung der Formel I oder deren entsprechendes Additionssalz man gewünschtenfalls in das cetal und/oder entsprechende Additionssalz überführt.

8. Verfahren nach Anspruch 7, dadurch gekennzeichnet daß $Z^2$ ein Wasserstoffatom, ein Alkalimetall oder eine Gruppe —MgX ist, wobei X ein Halogenatom ist.

9. Verfahren nach jedem der Ansprüche 7 und 8, dadurch gekennzeichnet, daß das Alkylierungsmittel ein N-acyliertes Amin ist (wobei die Acylgruppe von einer niedren Alkancarbonsäure abgeleitet ist) oder ein Anhydrid einer niederen Alkancarbonsäure ist.

10. Verfahren nach Anspruch 8, dadurch gekennzeichnet, daß das Acylierungsmittel das N-Formylpiperdin ist.

11. Verfahren nach jedem der Ansprüche 7 und 8, dadurch gekennzeichnet, daß das Alkoxy-Carbonylierungsmittel der Chlorameisensäurester der niederen Alkylgruppe ist.

12. Verfahren nach Anspruch 7, dadurch gekennzeichnet, daß man den Ester in das Amid durch Einwirkung von Ammoniak überführt.

13. Verfahren nach jedem der Ansprüche 7 und 8, dadurch gekennzeichnet, daß das Acylierungsmittel ein Orthoester der Formel

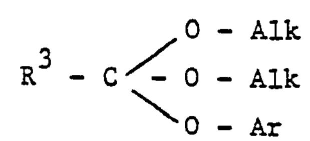

ist, wobei $R^3$ eine Gruppe aus —H oder eine niedere Alkylgruppe ist, —Alk eine niedere Alkylgruppe ist und Ar eine aromatische Gruppe wie eine Phenylgruppe ist, die aus einer entsprechenden Verbindung der Formel III erhalten worden ist, wobei $Z^3$ die Gruppe

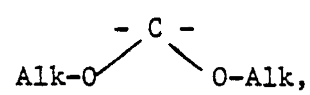

darstellt und man nach der Reaktion der Abspaltung der Schutzgruppe des Amins die Gruppe $Z^3$ gegebenenfalls in eine Carbonylgruppe durch Hydrolyse des Ketals nach bekannten Methoden überführt.

14. Verfahren nach jedem der Ansprüche 7 bis 13, dadurch gekennzeichnet, daß $Z^1$ eine Gruppe darstellt

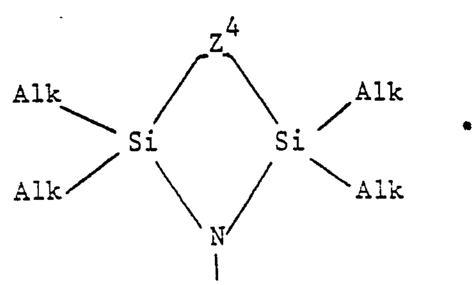

in der Alk eine niedere Alkylgruppe ist und $Z^4$ eine Alkylengruppe mit 2 oder 3 Kohlenstoffatomen in der Kette ist und daß man die Reaktion zur Abspaltung der Schutzgruppe des Amins durch saure Hydrolyse bewirkt.

15. Pharmazeutische Zubereitung, dadurch gekennzeichnet daß sie aus einem Wirkstoff bestehend aus mindestens einer Verbindung, ausgewählt aus Verbindung der Formel I

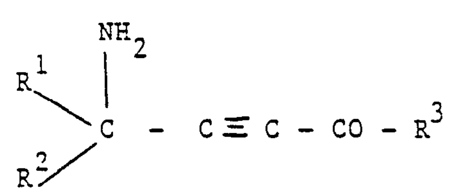

(I)

besteht, in der $R^1$ und $R^2$ jeweils unabhängig voneinander eine Alkylgruppe mit 1-5 Kohlenstoffatomen darstellt oder $R^1$ und $R^2$ zusammen eine Alkylengruppe mit 4 oder 5 Kohlenstoffatomen bedeuten und $R^3$ —H bedeutet oder eine niedere Alkylgruppe oder eine Gruppe —$NH_2$ ist, oder die entsprechenden Ketale und Additionssalze in Verbindungen der Formel I sowie Lösungen, die die Verbindungen oder deren Additionssalze enthalten.

16. Pharmazeutische Zubereitungen nach Anspruch 15, dadurch gekennzeichnet, daß sie einen Wirkstoff enthalten, derhaus mindestens einer Verbindung der Formel I A besteht :

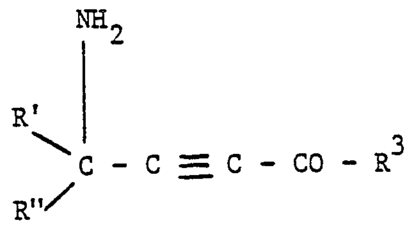

(IA)

in der R′ und $R^{2'}$ unabhängig voneinander eine Äthylgruppe oder eine Methylgruppe darstellen und $R^3$—H eine niedere Alkylgruppe oder $NH_2$ darstellen, oder mindestens ein Ketal und/oder ein Additionssalz der Verbindung I A. enthalten.

17. Zubereitungen nach Anspruch 15, dadurch gekennzeichnet, daß sie als Wirkstoff mindestens eine Verbindung der Formel

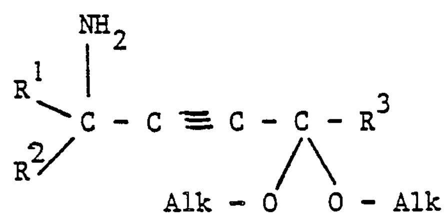

enthalten, wobei Alk eine niedere Alkylgruppe ist, $R^1$ und $R^2$ die oben genannte Bedeutung haben und $R^3$ —H oder eine niedere Alkylgruppe sind.

18. Zwischenprodukte, erhalten gemäß dem Verfahren nach einem der Ansprüche 7 bis 14, darstellend Verbindungen der Formel VI :

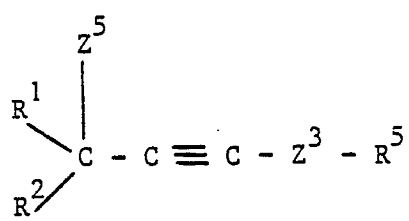

(VI)

in der entweder $Z^5$ eine geschützte Amingruppe darstellt und in dem Fall, daß $Z^3$ eine Carbonylgruppe oder eine Vorläufergruppe der Carbonylgruppe darstellt und $R^5$ —H oder eine niedere Alkylgruppe ist oder daß $Z^3$ eine Gruppe —CO— ist und $R^5$ eine niedere Alkylgruppe ist oder daß $Z^5$ eine Gruppe $NH_2$ ist und in dem Fall, daß $Z^3$ eine Carbonylgruppe und $R^5$ eine Gruppe —$OR^6$ darstellt, $R^6$ = —H, eine niedere Alkylgruppe oder eine Ammoniumgruppe oder das Äquivalent eines Metalls ist oder daß $Z^3$ eine geschützte Carbonylgruppe und $R^5$ —H oder eine niedere Alkylgruppe darstellt.

19. Zwischenprodukt nach Anspruch 16, bestehend aus Verbindungen der Formel VI, wobei $Z^5$ die folgende Bedeutung hat :

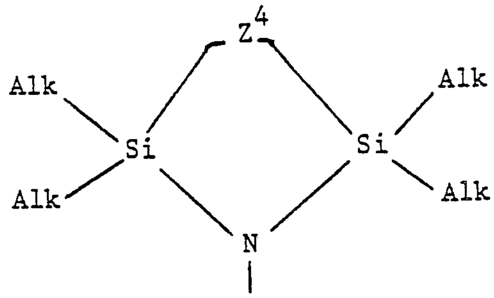

20. Zwischenprodukt nach Anspruch 18 oder 19, wobei Verbindungen der Formel VI eine Carbonyl-Vorläufer-Gruppe der folgenden Formel darstellen :

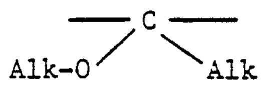 .